# FASCICULE DE BREVET EUROPEEN

(11) **EP 2 869 864 B1**
(45) Date de publication et mention de la délivrance du brevet: **08.06.2016**
(21) Numéro de dépôt: 13744688.6
(22) Date de dépôt: 04.07.2013
(51) Int. Cl.: A61M 1/02

(54) **APPAREIL POUR EXTRAIRE UN COMPOSANT SANGUIN CONTENU DANS UN SYSTÈME À POCHES**
VORRICHTUNG ZUR EXTRAKTION EINER BLUTKOMPONENTE IN EINEM SYSTEM AUS BEUTELN
APPARATUS FOR EXTRACTING A BLOOD COMPONENT CONTAINED IN A SYSTEM OF BAGS

(30) Priorité: 04.07.2012 FR 1256427
(43) Date de publication de la demande: 13.05.2015
(73) Titulaire: Maco Pharma, 59420 Mouvaux (FR); Delcon S.r.l., 20043 Arcore (IT)
(72) Inventeur: SCHROEDER, Tony, 63303 Dreieich (DE); RACANELLI, Massimo, 24052 Azzano San Paolo (BG) (IT)
(74) Mandataire: Sayettat, Julien Christian
(86) Numéro de dépôt international: PCT/FR2013/051592
(87) Numéro de publication internationale: WO 2014/006339

(56) Documents cités:
- EP-B1- 1 641 504
- DE-A1-102007 002 291
- FR-A1- 2 968 568
- US-A- 5 690 815
- US-A- 5 836 934
- US-A1- 2003 138 349

## Description

L'invention concerne un appareil pour extraire un composant sanguin contenu dans une poche primaire d'un système à poches, ainsi qu'un procédé réalisé à l'aide d'un tel appareil.

L'invention s'applique au domaine de la transfusion sanguine, en particulier au traitement du sang, et notamment la séparation des composants sanguins.

Le sang total est constitué de deux types de composants : les cellules sanguines comprenant les globules rouges, les leucocytes et les plaquettes, et le plasma dans lequel les cellules sanguines sont en suspension. Actuellement, ne sont transfusés que les composants sanguins nécessaires aux patients.

Aujourd'hui, dans les centres de transfusion ou les hôpitaux, ces différents composants sanguins sont séparés par centrifugation : le sang total d'un donneur est recueilli dans une poche dite poche primaire d'un système à poches. Puis, le système à poches est placé dans une centrifugeuse pour séparer les différents composants. Il existe deux types de centrifugation. La centrifugation dite douce du sang total conduit à le séparer en deux couches : une couche inférieure riche en globules rouges appelée concentré de globules rouges (CGR) ; et une couche supérieure contenant le plasma, les plaquettes et les leucocytes appelée plasma riche en plaquettes (PRP). La centrifugation dite dure conduit à une séparation en trois couches : une couche inférieure de CGR ; une couche supérieure de plasma pauvre en plaquettes (PPP) ; et une couche intermédiaire formée essentiellement de leucocytes et de plaquettes, dénommée couche leuco-plaquettaire ou buffy coat.

La poche primaire contenant les composants séparés par centrifugation est ensuite placée dans un appareil de séparation des composants par compression de la poche primaire, de manière que, par exemple, le plasma soit envoyé, via une tubulure, dans une première poche satellite. Les concentrés de globules rouges extraits sont en outre à additionner d'une solution additive de type SAGM (saline - adénine - glucose - mannitol) afin de pouvoir les conserver jusqu'à 42 jours.

Un exemple d'un appareil de séparation des composants sanguins est par exemple décrit dans le document EP 1 641 504. Cet appareil de séparation est notamment muni de clamps-soudeuses pour un fonctionnement quasi automatique. Cependant, avec ce type d'appareil, l'opérateur doit encore procéder à la rupture manuelle des ouvre-circuits du système à poches. En outre, cet appareil ne permet pas d'obtenir un mélange homogène du concentré de globules rouges et de solution additive.

Or, il est aussi connu que les leucocytes ont des effets indésirables très importants, ce qui a conduit à chercher à les éliminer des composants sanguins destinés à la transfusion. Ainsi, le mélange du concentré de globules rouges et de la solution additive est filtré au travers d'un filtre à déleucocyter afin d'éliminer le plus grand nombre de leucocytes possible. Mais, si le mélange n'est pas homogène, la filtration risque de ne pas être efficace.

On connaît également des documents DE 10 2007 002 291 et US 5 836 934, des appareils de séparation et filtration des composants sanguins par compression. Dans ces systèmes, le concentré de globules rouges et la solution additive sont envoyés dans une tubulure commune, puis le concentré de globules rouges additionné de solution additive est filtré et recueilli dans une poche de conservation des globules rouges. Ces systèmes d'addition et de mélange, s'ils sont suffisants pour réaliser une filtration en ligne sous pression du concentré de globules rouges, ne sont pas satisfaisants en terme de mélange, lorsque le concentré de globules rouges doit être filtré par gravité.

L'invention propose un appareil complètement automatisé permettant notamment l'extraction et le mélange homogène d'un concentré de globules rouges et de sa solution additive, afin de permettre une filtration optimale du concentré de globules rouges.

A cet effet, selon un premier aspect, l'invention concerne un appareil pour extraire au moins un composant sanguin contenu dans une poche primaire d'un système à poches, ledit système à poches comprenant en outre au moins une poche de solution contenant une solution additive pour ledit composant sanguin et au moins une poche satellite de recueil destinée à recueillir ledit composant sanguin additionné de ladite solution additive, lesdites poches étant associées entre elles par des tubulures, ledit appareil comprenant :
- un dispositif d'extraction destiné à extraire le composant sanguin par compression de la poche primaire,
- un dispositif de contrôle destiné à permettre le transfert dudit composant extrait ou dudit composant extrait et de la solution additive dans la poche satellite de recueil par l'intermédiaire des tubulures,
- un dispositif d'agitation destiné à agiter la poche satellite de recueil, et
- un système de pilotage pour commander ledit dispositif d'extraction, ledit dispositif de contrôle et ledit dispositif d'agitation, ledit système étant conçu pour permettre le mélange homogène du composant sanguin extrait et de la solution additive dans ladite poche satellite de recueil.

Selon un deuxième aspect, l'invention concerne un procédé pour extraire au moins un composant sanguin contenu dans une poche primaire d'un système à poches, à l'aide d'un appareil selon le premier aspect de l'invention, ledit procédé comprenant les étapes de :
- compresser la poche primaire de sorte à extraire le composant sanguin de la poche primaire et le transférer dans la poche satellite de recueil,
- transférer la solution additive de la poche de solution dans la poche satellite de recueil lors du transfert du composant sanguin extrait dans ladite poche satellite de recueil,
- agiter la poche satellite de recueil lors du transfert du composant sanguin extrait et de la solution additive dans ladite poche satellite de recueil.

D'autres objets et avantages apparaîtront au cours de la description qui suit.
La figure 1 représente de façon schématique un système à poches particulier utilisé avec un appareil selon l'invention.
La figure 2 représente de façon schématique une vue en perspective d'une première réalisation d'un appareil selon l'invention.
La figure 3 représente de façon schématique une vue partielle et en perspective d'une deuxième réalisation d'un appareil selon l'invention.
La figure 4 représente de façon schématique le fonctionnement d'un dispositif de rupture d'un élément de fermeture.
La figure 5 représente de façon schématique une vue partielle et en perspective d'un dispositif de rupture inclus dans un appareil selon l'invention.
Les figures 6 à 8 représentent de façon schématique une vue partielle et en éclaté d'un dispositif d'agitation d'un appareil selon une première réalisation de l'invention.
La figure 9 représente de façon schématique une vue partielle d'un dispositif d'agitation d'un appareil selon une deuxième réalisation de l'invention.
Les figures 10 et 11 représentent de façon schématique une vue partielle d'un dispositif d'agitation d'un appareil selon une troisième réalisation de l'invention.

Lors d'un don de sang, le sang est recueilli dans un système à poches comprenant plusieurs poches associées entre elles par des tubulures.

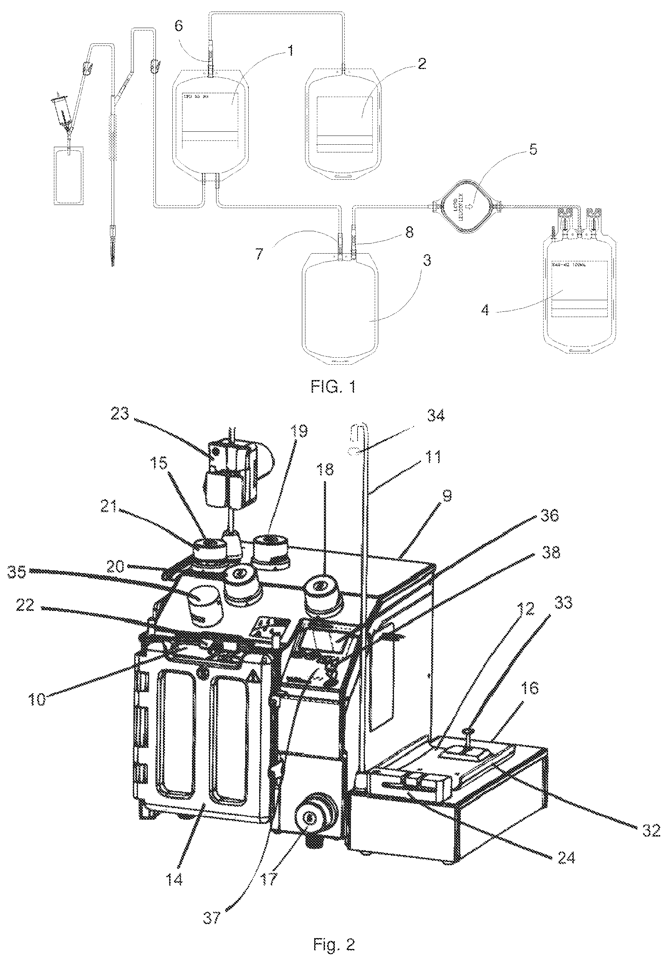

Un tel système à poches est par exemple représenté sur la figure 1 et comprend une poche primaire 1 destinée à recueillir le sang du donneur, une poche satellite 2 de recueil du plasma, une poche satellite 3 de recueil du concentré de globules rouges, et une poche de solution 4 contenant une solution additive. Une telle solution est par exemple la solution SAGM (saline-adénine-glucose-mannitol) permettant de conserver les concentrés de globules rouges jusqu'à 42 jours. La poche primaire 1 contient également un anticoagulant de type CPD (Citrate Phosphate Dextrose) ou ACD (Acide Citrate Dextrose).

Selon la figure 1, le système à poches comprend en outre une unité de filtration 5 pour déleucocyter les concentrés de globules rouges. Une telle unité de filtration est par exemple décrite dans le document EP 1 336 417.

Les différentes poches sont associées entre elles par des tubulures. Des éléments de fermeture 6,7,8 comportant une zone de fragilité, autrement appelés ouvre-circuits, sont logés dans les tubulures, à proximité des voies d'accès des poches. Ces éléments de fermeture empêchent l'écoulement des fluides dans les tubulures jusqu'à la rupture de la zone de fragilité. De tels éléments de fermeture sont décrits par exemple dans le document WO-93/17734. Un dispositif pour rompre de façon automatique ces éléments de fermeture est décrit dans le document FR 2 968 568.

En pratique, après avoir collecté le sang dans la poche primaire d'un tel système à poches, ce système à poches est placé dans une centrifugeuse afin de séparer les différents composants sanguins.

Après la centrifugation, la poche primaire 1 contient les différentes couches de composants sanguins, à savoir une couche de plasma, une couche de concentré de globules rouges et éventuellement une couche leuco-plaquettaire.

Afin d'extraire ces différents composants sanguins dans les poches appropriées 2,3, on utilise un appareil de séparation, qui, après rupture manuelle des éléments de fermeture 6,7,8 des tubulures, transfère automatiquement par un dispositif d'extraction par compression les différents composants sanguins dans les poches dédiées 2,3.

L'invention concerne un tel appareil destiné à extraire au moins un composant sanguin contenu dans une poche primaire 1 d'un système à poches, ledit système à poches comprenant au moins une poche de solution 4 contenant une solution additive pour ledit composant sanguin et au moins une poche satellite 3 de recueil destinée à recueillir ledit composant sanguin additionné de ladite solution additive, lesdites poches 1,3,4 étant associées entre elles par des tubulures.

Le composant sanguin est notamment un concentré de globules rouges, un plasma ou un concentré plaquettaire ou un pool de concentrés plaquettaires. Dans ce cas, la solution additive est notamment une solution de conservation. Un exemple de solution de conservation pour les plaquettes est la solution SSP+ de Maco Pharma (France). En variante, la solution additive est une solution contenant un agent pour inactiver les pathogènes du sang.

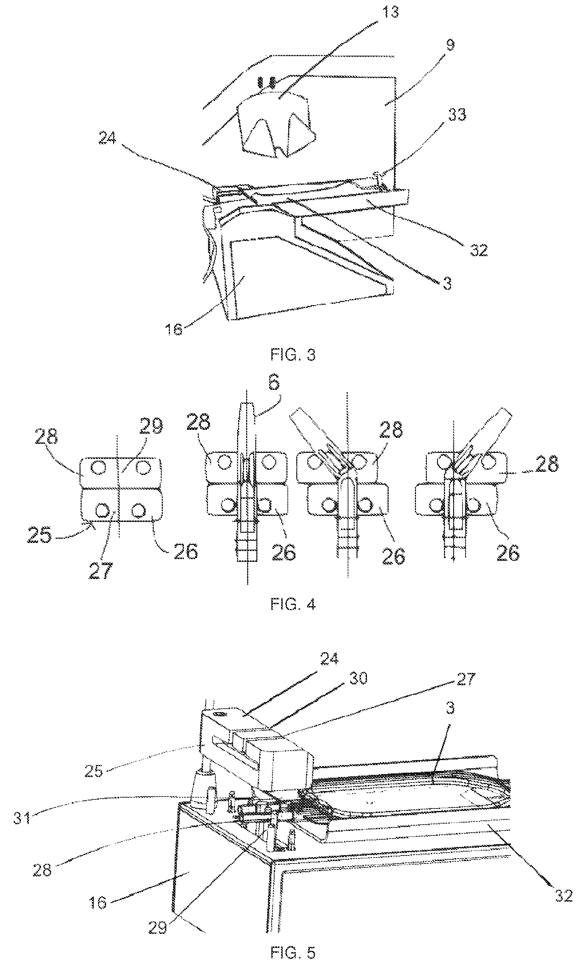

Selon les figures 2 et 3, l'appareil 9 se présente sous la forme d'un boîtier muni de supports 10,11,12 pour placer les différentes poches et unités de filtration du système à poches sur l'appareil. Ces dispositifs sont par exemple des crochets de suspension ou des plateaux.

Dans une réalisation particulière représentée sur la figure 3, le support 13 est destiné à supporter l'unité de filtration du système à poches (non représenté sur la figure 3) et se présente sous la forme de deux parois parallèles reliées entre elles par une paroi de fond courbe. La paroi de fond courbe présente une ouverture qui se prolonge sur l'une des parois parallèles. Cette ouverture est destinée à laisser passer la tubulure liée à l'unité de filtration.

L'appareil 9 comprend :
- un dispositif d'extraction 14 destiné à extraire le composant sanguin par compression de la poche primaire,
- un dispositif de contrôle 15 destiné à permettre le transfert dudit composant extrait ou dudit composant extrait et de la solution additive dans la poche satellite de recueil par l'intermédiaire des tubulures,
- un dispositif d'agitation 16 destiné à agiter la poche satellite de recueil, et
- un système de pilotage pour commander lesdits dispositif d'extraction, de contrôle et d'agitation, ledit système étant conçu pour permettre le mélange homogène du composant sanguin extrait et de la solution additive dans ladite poche satellite 3 de recueil.

Selon une réalisation, le dispositif d'extraction 14 comprend deux parois verticales et parallèles entre elles, entre lesquelles la poche primaire 1 est destinée à être placée. L'une au moins des parois est mobile en translation pour venir compresser la poche primaire 1 afin d'extraire les composants sanguins et les transférer dans les poches 2,3 satellites dédiées.

Le dispositif de contrôle 15 comprend un ensemble de clamps 17,18,19,20,21 pour les tubulures associant entre elles la poche primaire 1, la poche 4 de solution additive et la poche satellite 3 de recueil, respectivement.

Par exemple, le dispositif de contrôle 15 comprend entre un et six clamps. Chaque clamp est apte à pincer une tubulure pour bloquer l'écoulement fluidique à l'intérieur de ladite tubulure.

Notamment, les clamps permettent de contrôler l'écoulement des composants séparés provenant de la poche primaire 1, l'écoulement du plasma dans la poche satellite 2 de recueil du plasma, l'écoulement du concentré de globules rouges dans la poche satellite 3 de recueil du concentré de globules rouges et l'écoulement de la solution additive de la poche 4 de solution dans la poche satellite 3 de recueil du concentré de globules rouges.

Dans certains systèmes à poches, la solution additive est déjà présente dans la poche satellite 3 de recueil des concentrés de globules rouges. Dans ce cas, la poche satellite 3 de recueil des concentrés de globules rouges et la poche 4 de solution sont confondues. Le dispositif de contrôle 15 permet alors le transfert d'un composant extrait de la poche primaire 1 dans la poche satellite 3 de recueil par l'intermédiaire des tubulures.

Lorsque la solution additive est contenue dans une poche 4 de solution, distincte de la poche satellite 3 de recueil du composant, le dispositif de contrôle 15 permet le transfert du composant extrait de la poche primaire 1 et de la solution additive dans la poche satellite 3 de recueil du composant sanguin.

Dans un exemple particulier, les clamps 17,18,19,20,21 comprennent une soudeuse apte à souder la tubulure de sorte à fermer définitivement l'écoulement dans ladite tubulure, après extraction et transfert des composants sanguins séparés dans les poches appropriées.

Selon un autre exemple, l'un au moins des clamps 17,18,19,20,21 comprend un régulateur de débit. Ce clamp 20 muni d'un régulateur de débit permet notamment de contrôler la vitesse d'écoulement du plasma dans la poche de plasma. La régulation du débit de plasma assure le maintien de la couche leuco-plaquettaire dans une position sensiblement constante dans la poche primaire lors d'une extraction dite top&bottom, c'est-à-dire, lorsque le plasma et le concentré de globules rouges sont extraits simultanément de la poche primaire 1 par le haut et le bas de la poche primaire, respectivement.

Chaque clamp 17,18,19,20,21 est en outre muni d'un détecteur optique afin de signaler le bon positionnement de la tubulure dans le clamp.

Selon une autre réalisation, le dispositif de contrôle 15 comprend un ensemble de dispositifs 22,23,24 de rupture d'au moins un élément de fermeture comportant une zone de fragilité, ledit élément de fermeture étant disposé à l'intérieur de l'une des tubulures associant entre elles la poche primaire, la poche de solution additive et la poche satellite de recueil, respectivement.

Par exemple, le dispositif de contrôle comprend entre un et quatre dispositifs de rupture. Ces dispositifs de rupture automatisent le procédé d'extraction des composants sanguins.

Selon une réalisation particulière, l'appareil est muni de trois dispositifs 22,23,24 de rupture : l'un 22 destiné à rompre l'élément de fermeture de la poche primaire 1, le deuxième 23 pour la poche 4 de solution additive, et le troisième 24 pour la poche satellite 3 de recueil du concentré de globules rouges.

En variante, le dispositif 24 de rupture permet de rompre simultanément deux éléments de fermeture adjacents sur une poche.

Selon la figure 4, chacun desdits dispositifs 22-24 de rupture comporte un ensemble de réception 25 d'au moins une tubulure, ledit ensemble comprenant un élément fixe 26 pourvu d'un premier logement 27 destiné à recevoir une première portion de ladite tubulure, un élément mobile 28 pourvu d'un deuxième logement 29 destiné à recevoir une deuxième portion de ladite tubulure souple, et un organe d'entraînement en déplacement de l'élément mobile 28, de sorte à pouvoir provoquer la rupture de la zone de fragilité de l'élément de fermeture lorsque la tubulure est placée dans l'ensemble de réception 25.

Sur la figure 5, le dispositif de rupture 24 assure la rupture simultanée de deux éléments de fermeture d'une poche satellite 3 de recueil d'un composant sanguin, la poche comprenant deux vois d'accès disposées sur le haut de la poche. Dans cette réalisation, l'ensemble de réception 25 comprend un élément fixe 26 pourvu d'un premier logement 27 et d'un deuxième logement 30, et un élément mobile 28 pourvu d'un premier logement 29 et d'un deuxième logement 31 pour tubulures. Les logements 28,29 de l'élément mobile 28 sont sous forme de tiges.

Une description plus détaillée de tels dispositifs 22-24 de rupture se trouve dans le document FR 2968 568.

L'appareil comprend avantageusement un ensemble de clamps 17-21 et un ensemble de dispositifs 22-24 de rupture.

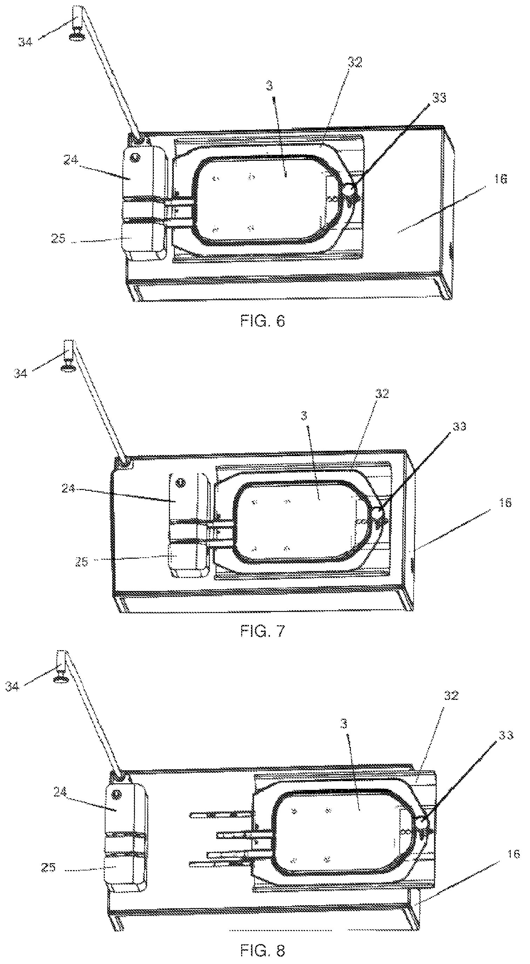

Selon l'invention et en relation avec les figures 6 à 11, l'appareil 9 comprend un dispositif d'agitation 16 destiné à agiter la poche satellite 3 de recueil du composant sanguin.

Le dispositif d'agitation 16 comprend un plateau mobile 32 sur lequel la poche satellite 3 de recueil est destinée à être placée. Le plateau 32 comprend notamment des moyens 33 de maintien de la poche sur le plateau, tel que par exemple une tige ou une vis, introduite dans un oeillet ou une fente de la poche.

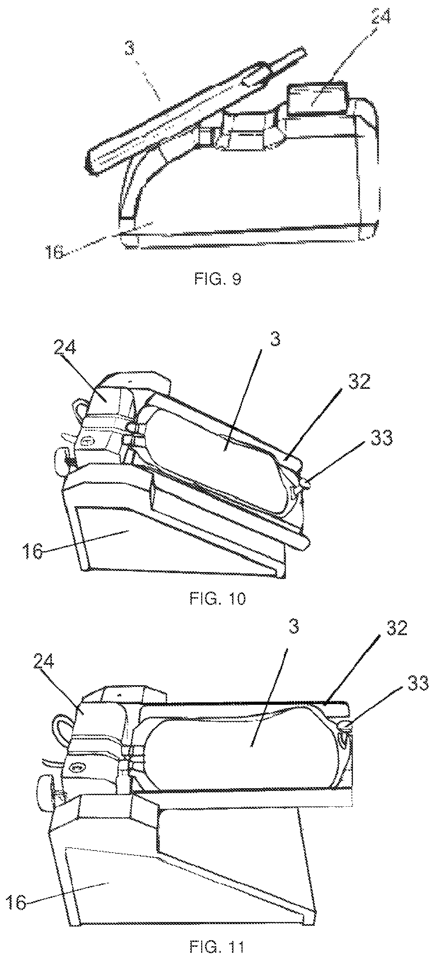

Ce plateau 32 est mobile soit selon un mouvement linéaire (figures 6 à 8) et/ou selon un mouvement de rotation (figures 9 à 11).

Lorsque le mouvement est linéaire, le mouvement du plateau 32 est un mouvement de va-et-vient, la poche satellite 3 de recueil étant placée à l'horizontal.

Avantageusement, le mouvement du plateau mobile 32 est un mouvement de rotation. Ce mouvement limite la longueur de tubulure nécessaire pour assurer le mouvement.

Selon la figure 9, l'axe de rotation du plateau mobile 32 est placé transversalement audit plateau, notamment à proximité ou sur l'axe transversal dudit plateau.

Avantageusement et comme représenté sur les figures 10 et 11, l'axe de rotation du plateau mobile 32 est placé transversalement audit plateau, à proximité de la partie avant du plateau destinée à recevoir le haut de la poche satellite 3 de recueil.

Dans le cas où le dispositif de contrôle 15 comprend un dispositif 24 de rupture, celui est associé au plateau mobile. Ainsi, la poche satellite 3 de recueil est maintenue en place sur le plateau par au moins ledit dispositif de rupture 24 (figure 7).

En variante, le dispositif de rupture 24 est fixe par rapport au plateau mobile 32 et le dispositif d'agitation 16 est agencé pour que la poche satellite 3 de recueil soit mobile d'une position dans laquelle l'élément de fermeture 7,8 de la tubulure associée à ladite poche est dans l'ensemble de réception 25 d'un dispositif de rupture 24 (figure 6) vers une position dans laquelle ledit élément de fermeture 7,8 est en dehors dudit ensemble de réception 25 (figure 8). Cette réalisation est plus simple à mettre en oeuvre et plus compacte.

Ce mouvement particulier du dispositif d'agitation 16 permet de libérer les éléments de fermeture de la poche satellite 3 de recueil du dispositif de rupture 24, avant de générer le mouvement du plateau 32.

Dans une autre variante particulièrement avantageuse représentée sur les figures 10 et 11, le dispositif de rupture 24 est fixe et le plateau 32 est mobile selon un mouvement de rotation dans lequel l'axe de rotation est placé transversalement audit plateau, à proximité de la partie avant du plateau 32 destinée à recevoir le haut de la poche satellite 3 de recueil, c'est-à-dire à proximité du dispositif de rupture 24. Ainsi, aucune réserve de longueur de tubulure n'est nécessaire pour assurer le mouvement de rotation et la poche est bien maintenue en place pendant le mouvement du plateau 32 par le dispositif de rupture 24.

Dans une réalisation particulière, l'appareil 9 comprend en outre un dispositif de pesage destiné à peser l'une poche au moins, ladite poche primaire, ladite poche satellite de recueil ou la poche de solution. Le dispositif de pesage comprend un ensemble de balances 34 de la poche primaire, de la poche satellite de recueil ou de la poche de solution, respectivement. Ce dispositif de pesage permet de contrôler d'une part la bonne séparation des composants et d'autre part la bonne rupture des éléments de fermeture. Par exemple, si un élément de fermeture d'une poche n'a pas été rompu correctement, l'écoulement de fluide vers ou de cette poche ne sera pas possible et le poids de la poche ne sera pas modifié.

L'appareil 9 comprend en outre un ensemble de détecteurs optiques 35 placés au niveau de la poche primaire 1 et/ou des tubulures afin de détecter l'interface entre les différents composants sanguins et/ou le passage d'un composant dans les tubulures.

L'appareil 9 est muni d'un système de pilotage pour le faire fonctionner. Le système de pilotage commande notamment lesdits dispositifs d'extraction 14, de contrôle 15 et d'agitation 16.

Le système de pilotage comprend notamment un microprocesseur conçu pour exécuter un programme de commandes. L'exécution de ce programme permet au système de pilotage de piloter les dispositifs d'extraction 14, de contrôle 15 et d'agitation 16, en fonction par exemple des signaux reçus par les détecteurs optiques et/ou le dispositif de pesage.

Le système de pilotage est conçu pour permettre le mélange homogène du composant sanguin extrait et de la solution additive dans ladite poche satellite 3 de recueil.

Par exemple, le système de pilotage est apte à commander les dispositifs d'extraction 14, de contrôle 15 et d'agitation 16, de sorte à transférer la solution additive dans la poche satellite 3 de recueil lors de l'extraction du composant sanguin et à agiter ladite poche satellite 3 de recueil lors du transfert du composant sanguin extrait et de la solution additive dans ladite poche satellite 3 de recueil.

En particulier, pour un système à poches tel que représenté sur la figure 1, le système de pilotage exécute les actions suivantes:
- fermeture des clamps 17,18,19,20,21,
- rupture des éléments de fermeture des différentes tubulures 6,7,8,
- ouverture des clamps 17,18,19,20,21 pour permettre l'extraction des composants sanguins dans les poches satellites 2,3 et l'écoulement de la solution additive dans la poche satellite 3 de recueil,
- compression de la poche primaire 1 contenant les composants sanguins préalablement séparés par centrifugation, la compression entraînant le transfert du plasma dans une poche satellite 2 de recueil du plasma et le transfert du concentré de globules rouges dans la poche satellite 3 de recueil des globules rouges,
- agitation de la poche satellite 3 de recueil.

Le système de pilotage est avantageusement conçu pour agiter la poche satellite 3 de recueil lors de la compression de la poche primaire 1 et/ou pour permettre le transfert simultané du composant sanguin et de sa solution additive dans la poche satellite 3 de recueil en contrôlant de façon appropriée l'ouverture des clamps 17-21 et la rupture des éléments de fermeture 6-8.

Ainsi, il est possible d'obtenir de façon automatisée un mélange homogène de composant sanguin et de solution additive en combinant une technique de mélange et une technique d'agitation. En effet, un premier mélange se produit en introduisant au moins en partie simultanément la solution additive et le composant sanguin dans la poche satellite de recueil. L'agitation à l'aide du dispositif d'agitation assure ensuite l'homogénéité du mélange.

L'appareil 9 comprend en outre une interface utilisateur 36 qui présente notamment un écran de visualisation 37 et un clavier 38.

Selon un deuxième aspect, l'invention concerne un procédé pour extraire au moins un composant sanguin contenu dans une poche primaire 1 d'un système à poches, à l'aide d'un appareil 9 selon le premier aspect de l'invention, ledit procédé comprenant les étapes de :
- compresser la poche primaire 1 de sorte à extraire le composant sanguin de la poche primaire et le transférer dans la poche satellite 3 de recueil,
- transférer la solution additive de la poche 4 de solution dans la poche satellite 3 de recueil lors du transfert du composant sanguin extrait dans ladite poche satellite 3 de recueil, et
- agiter la poche satellite 3 de recueil lors du transfert du composant sanguin extrait et de la solution additive dans ladite poche satellite 3 de recueil.

Notamment, le composant sanguin à extraire est un concentré de globules rouges obtenu par centrifugation d'une unité de sang total. En variante, le composant sanguin est un concentré de plaquettes obtenu par centrifugation d'un pool de couches leuco-plaquettaires.

La solution additive est une solution de conservation telles que celles décrites ci-dessus.

Le système à poches utilisé dans ce procédé comprend au moins une poche primaire 1, une poche 4 de solution contenant une solution additive pour ledit composant sanguin et au moins une poche satellite 3 de recueil destinée à recueillir ledit composant sanguin additionné de ladite solution additive, lesdites poches 1,3,4 étant distinctes et associées entre elles par des tubulures.

Selon le système à poches utilisé, la compression de la poche primaire 1 permet l'extraction des composants sanguins de façon consécutive (procédé top-top) ou simultanée (procédé top&bottom). Pour l'extraction consécutive des composants sanguins, la poche primaire 1 est munie de voie d'accès sur le haut de la poche. Pour l'extraction simultanée, la poche primaire 1 présente des voies d'accès sur le haut et le bas de la poche (figure 1).

Lors de la compression de la poche primaire 1 et le transfert du composant sanguin dans la poche satellite 3 de recueil, le procédé prévoit de transférer la solution additive dans ladite poche satellite 3 de recueil. Ce transfert, au moins en partie simultané, de la solution additive et du composant sanguin assure un mélange efficace des deux liquides.

Selon une réalisation, le transfert de la solution additive dans la poche satellite 3 de recueil est réalisé par gravité. Dans ce cas, la poche 4 de solution est simplement suspendue au dessus de la poche satellite 3 de recueil.

Avantageusement, une unité de filtration 5 est prévue sur la tubulure reliant la poche 4 de solution et la poche satellite 3 de recueil. Ainsi, lors du transfert de la solution additive dans la poche satellite 3 de recueil, la solution additive passe au travers de l'unité de filtration 5 disposée entre la poche 4 de solution et la poche satellite 3 de recueil.

Dans un système à poches particulier, la tubulure associant la poche primaire 1 et la poche satellite 3 de recueil et la tubulure associant la poche 4 de solution et la poche satellite 3 de recueil comprennent une portion de tubulure commune connectée par un connecteur en Y. Dans ce cas, le composant sanguin extrait et la solution additive sont mélangés l'un à l'autre dans ladite portion commune de tubulure.

En variante, la solution additive et le composant sanguin extrait sont transférés dans la poche satellite 3 de recueil par deux voies d'accès distinctes de ladite poche satellite 3 de recueil.

Le procédé selon l'invention prévoit d'agiter la poche satellite 3 de recueil lors du transfert du composant sanguin extrait et de la solution additive dans ladite poche satellite 3 de recueil. L'agitation est réalisée selon un mouvement linéaire et/ou de rotation.

Cette agitation améliore l'homogénéité du mélange de composant sanguin et de solution additive.

Avec le procédé de l'invention, le composant extrait et additionné de solution additive, peut ensuite être filtré au travers d'une unité de filtration 5, sans étape supplémentaire d'homogénéisation du mélange.

## Revendications

1. Appareil (9) pour extraire au moins un composant sanguin contenu dans une poche primaire (1) d'un système à poches, ledit système à poches comprenant en outre au moins une poche (4) de solution contenant une solution additive pour ledit composant sanguin et au moins une poche satellite (3) de recueil destinée à recueillir ledit composant sanguin additionné de ladite solution additive, lesdites poches (1,3,4) étant associées entre elles par des tubulures, ledit appareil comprenant :
- un dispositif d'extraction (14) destiné à extraire le composant sanguin par compression de la poche primaire (1), et
- un dispositif de contrôle (15) destiné à permettre le transfert dudit composant extrait ou dudit composant extrait et de la solution additive, dans la poche satellite (3) de recueil par l'intermédiaire des tubulures, **caractérisé en ce qu'**il comprend en outre :
- un dispositif d'agitation (16) destiné à agiter la poche satellite (3) de recueil, et
- un système de pilotage pour commander ledit dispositif d'extraction (14), ledit dispositif de contrôle (15) et ledit dispositif d'agitation (16), ledit système étant conçu pour permettre le mélange homogène du composant sanguin extrait et de la solution additive dans ladite poche satellite (3) de recueil.

2. Appareil selon la revendication 1, **caractérisé en ce que** le dispositif d'agitation (16) comprend un plateau mobile (32) sur lequel la poche satellite (3) de recueil est destinée à être placée.

3. Appareil selon la revendication 2, **caractérisé en ce que** le plateau (32) est mobile selon un mouvement linéaire.

4. Appareil selon l'une des revendications 2 ou 3, **caractérisé en ce que** le plateau (32) est mobile selon un mouvement en rotation.

5. Appareil selon la revendication 4, **caractérisé en ce que** l'axe de rotation du plateau mobile (32) est placé transversalement audit plateau (32).

6. Appareil selon la revendication 5, **caractérisé en ce que** l'axe de rotation du plateau mobile (32) est placé à proximité de la partie avant du plateau (32) destinée à recevoir le haut de la poche satellite (3) de recueil.

7. Appareil selon l'une des revendications 1 à 6, **caractérisé en ce que** le dispositif de contrôle (15) comprend un ensemble de clamps (17,18,19,20,21) pour les tubulures associant entre elles la poche primaire (1), la poche (4) de solution additive et la poche satellite (3) de recueil, respectivement.

8. Appareil selon l'une des revendications 1 à 7, **caractérisé en ce que** le dispositif de contrôle (15) comprend un ensemble de dispositifs de rupture (22,23,24) d'au moins un élément de fermeture (6,7,8) comportant une zone de fragilité, ledit élément de fermeture étant disposé à l'intérieur de l'une des tubulures associant entre elles la poche primaire (1), la poche (4) de solution additive et la poche satellite (3) de recueil, respectivement.

9. Appareil selon la revendication 8, **caractérisé en ce que** chacun desdits dispositifs de rupture (22,23,24) comporte un ensemble de réception (25) d'au moins une tubulure, ledit ensemble comprenant un élément fixe (26) pourvu d'un premier logement (27) destiné à recevoir une première portion de ladite tubulure, un élément mobile (28) pourvu d'un deuxième logement (29) destiné à recevoir une deuxième portion de ladite tubulure souple, et un organe d'entraînement en déplacement de l'élément mobile, de sorte à pouvoir provoquer la rupture de la zone de fragilité de l'élément de fermeture lorsque la tubulure est placée dans l'ensemble de réception.

10. Appareil selon la revendication 9, **caractérisé en ce que** le dispositif d'agitation (16) est agencé pour que la poche satellite (3) de recueil soit mobile d'une position dans laquelle l'élément de fermeture (7,8) de la tubulure associée à ladite poche est dans l'ensemble de réception (25) d'un dispositif de rupture (24) vers une position dans laquelle ledit élément de fermeture (7,8) est en dehors dudit ensemble de réception (25).

11. Appareil selon l'une des revendications 1 à 10, **caractérisé en ce qu'**il comprend un dispositif de pesage (34) destiné à peser l'une au moins de la poche primaire, la poche satellite de recueil et la poche de solution.

12. Appareil selon l'une des revendications 1 à 11, **caractérisé en ce que** le système de pilotage est apte à commander les dispositifs d'extraction (14), de contrôle (15) et d'agitation (16), de sorte à transférer la solution additive dans la poche satellite (3) de recueil lors de l'extraction du composant sanguin et à agiter ladite poche satellite (3) de recueil lors du transfert du composant sanguin extrait et de la solution additive dans ladite poche satellite (3) de recueil.

13. Procédé pour extraire au moins un composant sanguin contenu dans une poche primaire (1) d'un système à poches, à l'aide d'un appareil selon l'une des revendications 1 à 12, ledit procédé comprenant les étapes de :
- compresser la poche primaire (1) de sorte à extraire le composant sanguin de la poche primaire (1) et le transférer dans la poche satellite (3) de recueil,
- transférer la solution additive de la poche de solution dans la poche satellite (3) de recueil lors du transfert du composant sanguin extrait dans ladite poche satellite (3) de recueil, ledit procédé étant **caractérisé en ce qu'**il comprend en outre l'étape d'agiter la poche satellite (3) de recueil lors du transfert du composant sanguin extrait et de la solution additive dans ladite poche satellite (3) de recueil.

14. Procédé selon la revendication 13, **caractérisé en ce que** le transfert de la solution additive dans la poche satellite (3) de recueil est réalisé par gravité.

15. Procédé selon l'un des revendications 13 ou 14, **caractérisé en ce que**, lors du transfert de la solution additive dans la poche satellite (3) de recueil, la solution additive passe au travers d'une unité de filtration (5) disposée entre la poche (4) de solution et la poche satellite (3) de recueil.

16. Procédé selon l'une des revendications 13 à 15, **caractérisé en ce que** la solution additive et le composant sanguin extrait sont transférés dans la poche satellite (3) de recueil par deux voies d'accès distinctes de ladite poche satellite (3) de recueil.

17. Procédé selon l'une des revendications 13 à 16, **caractérisé en ce que** l'agitation de la poche satellite (3) de recueil est réalisée selon un mouvement linéaire.

18. Procédé selon l'une des revendications 13 à 17, **caractérisé en ce que** l'agitation de la poche satellite (3) de recueil est réalisée selon un mouvement de rotation.

19. Procédé selon l'une des revendications 13 à 18, **caractérisé en ce que** le composant sanguin est un concentré de globules rouges obtenu par centrifugation d'une unité de sang total.

## Patentansprüche

1. Gerät (9) zum Extrahieren mindestens einer Blutkomponente, die in einem Primärbeutel (1) eines Systems mit Beuteln enthalten ist, wobei das System mit Beuteln außerdem mindestens einen Lösungsbeutel (4) umfasst, der eine Zusatzlösung für die Blutkomponente enthält, und mindestens einen Satellitenbeutel (3) zum Sammeln, der dazu bestimmt ist, die Blutkomponente, zu der die Zusatzlösung hinzugefügt ist, zu sammeln, wobei die Beutel (1, 3, 4) untereinander durch Anschlussstutzen verbunden sind, wobei das Gerät Folgendes umfasst:
- eine Extraktionsvorrichtung (14), die dazu bestimmt ist, die Blutkomponente durch Komprimieren des Primärbeutels (1) zu extrahieren, und
- eine Prüfvorrichtung (15), die dazu bestimmt ist, den Transfer der extrahierten Komponente oder der extrahierten Komponente und der Zusatzlösung in den Satellitenbeutel (3) zum Sammeln über Anschlussstutzen zu erlauben, **dadurch gekennzeichnet, dass** sie ferner Folgendes umfasst:
- eine Schüttelvorrichtung (16), die dazu bestimmt ist, den Satellitenbeutel (3) zum Sammeln zu schütteln, und
- ein Steuersystem, um die Extraktionsvorrichtung (14), die Steuervorrichtung (15) und die Schüttelvorrichtung (16) zu zu steuern, wobei das System konzipiert ist, um das homogene Mischen der extrahierten Blutkomponente und der Zusatzlösung in dem Satellitenbeutel (3) zum Sammeln zu erlauben.

2. Gerät nach Anspruch 1, **dadurch gekennzeichnet, dass** die Schüttelvorrichtung (16) eine bewegliche Platte (32) umfasst, auf der der Satellitenbeutel (3) zum Sammeln platziert werden soll.

3. Gerät nach Anspruch 2, **dadurch gekennzeichnet, dass** die Platte (32) entlang einer linearen Bewegung beweglich ist.

4. Gerät nach einem der Ansprüche 2 oder 3, **dadurch gekennzeichnet, dass** die Platte (32) gemäß einer Bewegung in Drehung beweglich ist.

5. Gerät nach Anspruch 4, **dadurch gekennzeichnet, dass** die Rotationsachse der beweglichen Platte (32) quer zu der Platte (32) platziert ist.

6. Gerät nach Anspruch 5, **dadurch gekennzeichnet, dass** die Rotationsachse der beweglichen Platte (32) in der Nähe des Vorderteils der Platte (32) platziert ist, der dazu bestimmt ist, die Oberseite des Satellitenbeutels (3) zum Sammeln aufzunehmen.

7. Gerät nach einem der Ansprüche 1 bis 6, **dadurch gekennzeichnet, dass** die Steuervorrichtung (15) eine Einheit von Klemmen (17, 18, 19, 20, 21) für die Anschlussstutzen, die jeweils den Hauptbeutel (1), den Beutel (4) mit Zusatzlösung und den Satellitenbeutel (3) zum Sammeln miteinander verbinden.

8. Gerät nach einem der Ansprüche 1 bis 7, **dadurch gekennzeichnet, dass** die Steuervorrichtung (15) eine Einheit von Vorrichtungen zum Brechen (22, 23, 24) mindestens eines Verschlusselements (6, 7, 8), das eine Zerbrechlichkeitszone umfasst, umfasst, wobei das Verschlusselement im Inneren eines der Anschlussstutzen, die jeweils den Primärbeutel (1), den Beutel (4) mit Zusatzlösung und den Satellitenbeutel (3) zum Sammeln miteinander verbinden, angeordnet ist.

9. Gerät nach Anspruch 8, **dadurch gekennzeichnet, dass** jede der Bruchvorrichtungen (22, 23, 24) eine Aufnahmeeinheit (25) mindestens eines Anschlussstutzens umfasst, wobei die Einheit ein stationäres Element (26) umfasst, das mit einer ersten Aufnahme (27) versehen ist, die dazu bestimmt ist, einen ersten Abschnitt des Anschlussstutzens aufzunehmen, ein bewegliches Element (28), das mit einer zweiten Aufnahme (29) versehen ist, die dazu bestimmt ist, einen zweiten Abschnitt des biegsamen Anschlussstutzens aufzunehmen, und ein Element zum Antreiben in Bewegung des beweglichen Elements derart, dass der Bruch der Zerbrechlichkeitszone des Verschlusselements verursacht wird, wenn der Anschlussstutzen in die Aufnahmeeinheit platziert wird.

10. Gerät nach Anspruch 9, **dadurch gekennzeichnet, dass** die Schüttelvorrichtung (16) eingerichtet ist, damit der Satellitenbeutel (3) zum Sammeln von einer Position, in der das Verschlusselement (7, 8) des Anschlussstutzens, das zu dem Beutel gehört, in der Aufnahmeeinheit (25) einer Bruchvorrichtung (24) ist, zu einer Position, in der das Verschlusselement (7, 8) außerhalb der Aufnahmeeinheit (25) ist, beweglich ist.

11. Gerät nach einem der Ansprüche 1 bis 10, **dadurch gekennzeichnet, dass** es eine Wiegevorrichtung (34) umfasst, die dazu bestimmt ist, den Primärbeutel und/oder den Satellitenbeutel zum Aufnehmen und/oder den Lösungsbeutel abzuwiegen.

12. Gerät nach einem der Ansprüche 1 bis 11, **dadurch gekennzeichnet, dass** das Steuersystem geeignet ist, die Extraktionsvorrichtung (14), Steuervorrichtung (15) und Schüttelvorrichtung (16) derart zu steuern, dass die Zusatzlösung in den Satellitenbeutel (3) zum Sammeln beim Extrahieren der Blutkomponente transferiert wird, und der Satellitenbeutel (3) zum Sammeln bei dem Transfer der extrahierten Blutkomponente und der Zusatzlösung in dem Satellitenbeutel (3) zum Sammeln geschüttelt wird.

13. Verfahren zum Extrahieren mindestens einer Blutkomponente, die in einem Primärbeutel (1) eines Systems mit Beuteln enthalten ist, mit Hilfe eines Geräts nach einem der Ansprüche 1 bis 12, wobei das Verfahren die folgenden Schritte umfasst:
- Komprimieren des Primärbeutels (1) derart, dass die Blutkomponente aus dem Primärbeutel (1) extrahiert und in den Satellitenbeutel (3) zum Sammeln transferiert wird,
- Transferieren der Zusatzlösung aus dem Lösungsbeutel in den Satellitenbeutel (3) zum Sammeln bei dem Transfer der extrahierten Blutkomponente in den Satellitenbeutel (3) zum Sammeln, Verfahren **dadurch gekennzeichnet, dass** es außerdem den Schritt des Schüttelns des Satellitenbeutels (3) zum Sammeln bei dem Transfer der extrahierten Blutkomponente und der Zusatzlösung in den Satellitenbeutel (3) zum Sammeln umfasst.

14. Verfahren nach Anspruch 13, **dadurch gekennzeichnet, dass** der Transfer der Zusatzlösung in den Satellitenbeutel (3) zum Sammeln durch Schwerkraft erfolgt.

15. Verfahren nach einem der Ansprüche 13 oder 14, **dadurch gekennzeichnet, dass** bei dem Transfer der Zusatzlösung in den Satellitenbeutel (3) zum Sammeln die Zusatzlösung durch eine Filtereinheit (5), die zwischen dem Lösungsbeutel (4) und dem Satellitenbeutel (3) zum Sammeln angeordnet ist, durchgeht.

16. Verfahren nach einem der Ansprüche 13 bis 15, **dadurch gekennzeichnet, dass** die Zusatzlösung und die extrahierte Blutkomponente in den Satellitenbeutel (3) zum Sammeln über zwei getrennte Zugangskanäle des Satellitenbeutels (3) zum Sammeln transferiert werden.

17. Verfahren nach einem der Ansprüche 13 bis 16, **dadurch gekennzeichnet, dass** das Schütteln des Satellitenbeutels (3) zum Sammeln entlang einer linearen Bewegung ausgeführt wird.

18. Verfahren nach einem der Ansprüche 13 bis 17, **dadurch gekennzeichnet, dass** das Schütteln des Satellitenbeutels (3) zum Sammeln entlang einer Rotationsbewegung ausgeführt wird.

19. Verfahren nach einem der Ansprüche 13 bis 18, **dadurch gekennzeichnet, dass** die Blutkomponente ein Konzentrat roter Blutkörperchen ist, das durch Zentrifugieren einer Vollbluteinheit erhalten wird.

## Claims

1. Apparatus (9) for extracting at least one blood component contained in a primary bag (1) of a system of bags, said system of bags further comprising at least one bag (4) of solution containing an additive solution for said blood component and at least one collecting satellite bag (3) intended for collecting said blood component to which said additive solution has been added, said bags (1, 3, 4) being connected to one another by tubes, said apparatus comprising:
- an extraction device (14) intended for extracting the blood component by compressing the primary bag (1), and
- a control device (15) intended for enabling the transfer of said extracted component or of said extracted component and of the additive solution, to the collecting satellite bag (3) by the intermediary of tubes, **characterised in that** it further comprises:
- an agitation device (16) intended for agitating the collecting satellite bag (3), and
- a drive system intended for commanding said extraction device (14), said control device (15) and said agitation device (16), said system being designed to enable the uniform mixing of the extracted blood component and of the additive solution in said collecting satellite bag (3).

2. Apparatus according to claim 1, **characterised in that** the agitation device (16) comprises a mobile plate (32) whereon the collecting satellite bag (3) is intended to be placed.

3. Apparatus according to claim 2, **characterised in that** the plate (32) is mobile according to a linear movement.

4. Apparatus according to one of claims 2 or 3, **characterised in that** the plate (32) is mobile according to a rotation movement.

5. Apparatus according to claim 4, **characterised in that** the axis of rotation of the mobile plate (32) is placed transversally to said plate (32).

6. Apparatus according to claim 5, **characterised in that** the axis of rotation of the mobile plate (32) is placed in the vicinity of the front portion of the plate (32) intended to receive the top of the collecting satellite bag (3).

7. Apparatus according to one of claims 1 to 6, **characterised in that** the control device (15) comprises a set of clamps (17, 18, 19, 20, 21) for the tubes connecting together the primary bag (1), the bag (4) of additive solution and the collecting satellite bag (3), respectively.

8. Apparatus according to one of claims 1 to 7, **characterised in that** the control device (15) comprises a set of devices for breaking (22, 23, 24) of at least one closing element (6, 7, 8) comprising a weak zone, said closing element being arranged inside one of the tubes connecting together the primary bag (1), the bag (4) of additive solution and the collecting satellite bag (3), respectively.

9. Apparatus according to claim 8, **characterised in that** each one of said devices for breaking (22, 23, 24) comprises a set for receiving (25) of at least one tube, said set comprising a fixed element (26) provided with a first housing (27) intended to receive a first portion of said tube, a mobile element (28) provided with a second housing (29) intended to receive a second portion of said flexible tube, and a member for driving the mobile element in displacement, in such a way as to be able to cause the breaking of the weak zone of the closing element when the tube is placed in the set for receiving.

10. Apparatus according to claim 9, **characterised in that** the agitation device (16) is arranged so that the collecting satellite bag (3) is mobile from a position wherein the closing element (7, 8) of the tube connected to said bag is in the set for receiving (25) of a device for breaking (24) to a position wherein said closing element (7, 8) is outside of said set for receiving (25).

11. Apparatus according to one of claims 1 to 10, **characterised in that** it comprises a weighing device (34) intended to weigh at least one of the primary bag, the collecting satellite bag and the bag of solution.

12. Apparatus according to one of claims 1 to 11, **characterised in that** the drive system is able to command the extraction (14), control (15) and agitation (16) devices, in such a way as to transfer the additive solution to the collecting satellite bag (3) during the extraction of the blood component and to agitate said collecting satellite bag (3) during the transfer of the extracted blood component and of the additive solution to said collecting satellite bag (3).

13. Method for extracting at least one blood component contained in a primary bag (1) of a system of bags, using an apparatus according to one of claims 1 to 12, said method comprising the steps of:
- compressing the primary bag (1) in such a way as to extract the blood component from the primary bag (1) and transfer it to the collecting satellite bag (3),
- transferring the additive solution from the bag of solution to the collecting satellite bag (3) during the transfer of the extracted blood component in said collecting satellite bag (3), said method being **characterised in that** it further comprises the step of agitating the collecting satellite bag (3) during the transfer of the extracted blood component and of the additive solution in said collecting satellite bag (3).

14. Method according to claim 13, **characterised in that** the transfer of the additive solution in the collecting satellite bag (3) is carried out via gravity.

15. Method according to one of claims 13 or 14, **characterised in that**, during the transfer of the additive solution to the collecting satellite bag (3), the additive solution passes through a filtration unit (5) arranged between the bag (4) of solution and the collecting satellite bag (3).

16. Method according to one of claims 13 to 15, **characterised in that** the additive solution and the extracted blood component are transferred to the collecting satellite bag (3) by two separate access channels of said collecting satellite bag (3).

17. Method according to one of claims 13 to 16, **characterised in that** the agitation of the collecting satellite bag (3) is carried out according to a linear movement.

18. Method according to one of claims 13 to 17, **characterised in that** the agitation of the collecting satellite bag (3) is carried out according to a rotation movement.

19. Method according to one of claims 13 to 18, **characterised in that** the blood component is a packed red blood cells obtained via centrifugation of a unit of whole blood.
